(19) Europäisches Patentamt
European Patent Office
Office européen des brevets

(11) **EP 3 263 023 A1**

(12) **EUROPEAN PATENT APPLICATION**
published in accordance with Art. 153(4) EPC

(43) Date of publication:
**03.01.2018 Bulletin 2018/01**

(51) Int Cl.:
***A61B 5/022*** (2006.01)  ***A61B 5/02*** (2006.01)
***A61B 5/0245*** (2006.01)

(21) Application number: **16755610.9**

(22) Date of filing: **25.02.2016**

(86) International application number:
**PCT/JP2016/055588**

(87) International publication number:
**WO 2016/136866 (01.09.2016 Gazette 2016/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **27.02.2015 JP 2015038728**

(71) Applicants:
• **Omron Healthcare Co., Ltd.
Muko-shi, Kyoto 617-0002 (JP)**

• **Omron Corporation
Kyoto-shi, Kyoto 600-8530 (JP)**

(72) Inventors:
• **KITAGAWA, Tsuyoshi
Muko-shi, Kyoto 617-0002 (JP)**
• **YAMASHITA, Shingo
Muko-shi, Kyoto 617-0002 (JP)**

(74) Representative: **Isarpatent
Patent- und Rechtsanwälte Behnisch Barth
Charles
Hassa Peckmann & Partner mbB
Friedrichstrasse 31
80801 München (DE)**

(54) **BIOLOGICAL INFORMATION MEASURING DEVICE**

(57) Provided are a control unit 12 for measuring living body information on the basis of pressure pulse waves detected by pressure sensors 6a and 7a in a state that the pressure sensors 6a and 7a are pressed toward a radius artery T by an air bag 2 and display units 104 and 105 which are arranged around a wrist in the state that the pressure sensors 6a and 7a are pressed toward the radius artery T. The control unit 12 performs display controls on the display units 104 and 105. The display unit 104 is disposed on the palm side of the wrist in the state that the pressure sensors 6a and 7a are pressed toward the radius artery T. The display unit 105 is disposed on the hand back side of the wrist in the state that the pressure sensors 6a and 7a are pressed toward the radius artery T.

*FIG. 1*

EP 3 263 023 A1

**Description**

**TECHNICAL FIELD**

The present invention relates to living body information measuring instrument.

**BACKGROUND ART**

Living body information measuring instruments are known which can measure living body information such as a pulse rate and a blood pressure continuously (for each beat) using information that is detected by a pressure sensor in a state that the pressure sensor is in direct contact with a living body part where an artery such as a wrist radius artery runs (refer to Patent documents 1-5).

In the blood pressure measuring instrument disclosed in Patent document 1, blood pressure values in a part that is different from a living body part to which a pressure sensor is brought into contact are calculated using a cuff and calibration data is generated using the calculated blood pressure values. Blood pressure values are calculated for each beat by calibrating a pressure pulse wave detected by the pressure sensor using the calibration data.

Patent documents 2, 3, and 4 disclose blood pressure measuring instruments which measure a blood pressure for each beat without using a cuff, that is, using only information detected by a pressure sensor that is set in contact with a wrist.

The living body information measuring instrument disclosed in Patent document 5 is of a wrist watch type and is configured such that a display unit is located on the palm side in a state that it is attached to a wrist. Living body information (exercise strength, pulse rate, etc.) that has been measured on the basis of pressure pulse waves detected by the pressure sensor are displayed on the display unit.

Patent document 6 discloses a blood pressure measuring instrument that is not of such a type that a pressure sensor is brought into contact with a living body part but a wrist watch type blood pressure measuring instrument capable of measuring a blood pressure by exerting pressure on a wrist using a cuff. This blood pressure measuring instrument is configured such that in a state that it is attached to a wrist a display unit is located on the back side of the hand and measured blood pressure values etc. are displayed on it.

**PRIOR ART DOCUMENTS**

**PATENT DOCUMENTS**

PATENT DOCUMENT 1: JP-2004-113368-A
PATENT DOCUMENT 2: JP-H02-261421-A

PATENT DOCUMENT 3: JP-H07-124130-A
PATENT DOCUMENT 4: JP-H01-242031-A
PATENT DOCUMENT 5: JP-2010-220949-A
PATENT DOCUMENT 6: WO-2012-018029-A

**SUMMARY OF THE INVENTION**

**PROBLEMS TO BE SOLVED BY THE INVENTION**

In living body information measuring instruments that are used being attached to a wrist as disclosed in Patent documents 5 and 6, inconvenience may occur if a display unit is located only the back side or the palm side of the hand.

For example, assume a case that a living body information measuring instrument is used in a hospital or the like and a health care professional is going to check living body information displayed on a display unit of the living body information measuring instrument that is attached to a wrist of a patient. The patient may not be able to move his or her hand in a desired manner depending on his or her physical condition, disease-related condition, or the like. In such a situation, it is necessary for the health care professional to check a display on the display unit by moving the wrist of the patient, which may be burdensome to both of the health care professional and the patient. Furthermore, where the patient is in a situation that he or she cannot move his or her hand in a desired manner, it is difficult to the patient to check information on the display unit.

For certain humans, it is more difficult to do an action for checking information on a display unit that is located on the palm side of a hand than on a display unit that is located on the back side of a hand. On the other hand, if a display unit is provided only on the back side of a hand, the display unit located there is easy to see to others, resulting is difficulty preserving his or her privacy.

The present invention has been made in view of the above circumstances, and an object of the invention is therefore to provide a living body information measuring instrument which allows both of a measurement-subject person and a third person to check measured living body information easily by giving a higher degree of freedom to the manner of display of measured living body information and enables consideration for preservation of the privacy of the measurement-subject person.

**MEANS FOR SOLVING THE PROBLEMS**

A living body information measuring unit according to the invention is a wrist-mounted living body information measuring instrument having a living body information measuring unit for measuring living body information on the basis of a living body signal detected from a wrist, including a main body which forms a space into which the wrist can be inserted; a first display unit and a second display unit which are provided on an outer circumference of the main body; and a display control unit which performs display controls on the first display unit and the second display unit, wherein the first display unit and the second display unit are disposed such that the space exists on a straight line that connects a center of a display screen of the first display unit and a center of a display screen of the second display unit, and a center of the space is located between the center of the display screen of the first display unit and the center of the display screen of the second display unit in a direction that is perpendicular to a palm in a state that the living body information measuring instrument is attached to the wrist.

## ADVANTAGES OF THE INVENTION

The invention can provide a living body information measuring instrument which allows both of a measurement-subject person and a third person to check measured living body information easily by giving a higher degree of freedom to the manner of display of measured living body information and enables consideration for preservation of the privacy of the measurement-subject person.

## BRIEF DESCRIPTION OF THE DRAWINGS

Fig. 1 is an appearance view showing the configuration of a blood pressure measuring instrument 200 for description of an embodiment of the present invention.
Fig. 2 is a view, as viewed from the palm side, of the blood pressure measuring instrument 200 of Fig. 1 being attached to a wrist.
Fig. 3 is a view, as viewed from the hand back side, of the blood pressure measuring instrument 200 of Fig. 1 being attached to the wrist.
Fig. 4 is an appearance view showing the configuration of a pressure pulse wave detection unit 100 of the blood pressure measuring instrument 200 shown in Fig. 1.
Fig. 5 is an enlarged view of the pressure pulse wave detection unit 100 shown in Fig. 4.
Fig. 6 is a view, as viewed from the side of the fingertips of the user, of the pressure pulse wave detection unit 100 shown in Fig. 4 that is in an attached state.
Fig. 7 is a view, as viewed from the side of the part being in contact with the wrist, of the pressure pulse wave detection unit 100 shown in Fig. 4 that is in an attached state.
Fig. 8 shows a block configuration of the blood pressure measuring instrument other than the pressure pulse wave detection unit 100.
Fig. 9 is a flowchart illustrating an operation, to generation of calibration data in a continuous blood pressure measurement mode, of the pressure measuring instrument according to the embodiment.
Figs. 10A and 10B are diagrams for showing an example state that a radius artery is not closed by one of two sensor portions.
Figs. 11A and 11B are diagrams for showing examples of amplitude values of pressure pulse waves that are detected by pressure sensors of a sensor unit 6 as the pressing force that the sensor unit 6 exerts on the wrist is varied.
Figs. 12A to 12C are diagrams for showing how pressing of the sensor unit 6 against the wrist by an air bag 2 proceeds after attachment of the pressure pulse wave detection unit 100 to the wrist.
Fig. 13 is a graph showing an example of how a pressure pulse wave detected by an optimum pressure sensor varies as the pressure acting on the wrist is varied.
Fig. 14 shows example pulse wave envelope data.
Fig. 15 is a flowchart illustrating a continuous blood pressure measuring operation in the continuous blood pressure measurement mode of the blood pressure measuring instrument according to the embodiment.
Fig. 16 shows example displays to be made on the screens of display units 104 and 105 of the blood pressure measuring instrument 200 shown in Fig. 1.
Fig. 17 shows other example displays to be made on the screens of display units 104 and 105 of the blood pressure measuring instrument 200 shown in Fig. 1.
Fig. 18 shows a modification (modified in terms of appearance) of the blood pressure measuring instrument 200 shown in Fig. 1.

## MODES FOR CARRYING OUT THE INVENTION

An embodiment of the present invention will be hereinafter described with reference to the drawings.

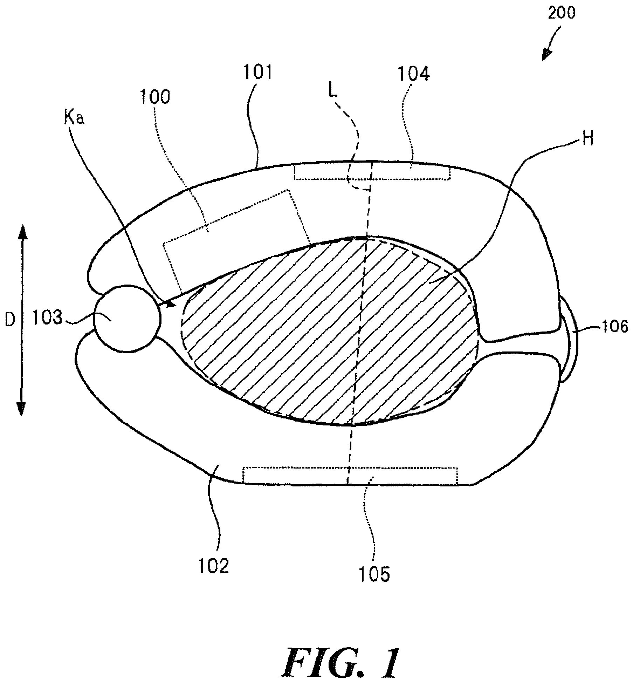

Fig. 1 is a side view showing the configuration of a blood pressure measuring instrument 200 which is an example living body information measuring instrument for description of the embodiment of the invention. The blood pressure measuring instrument 200 shown in Fig. 1 is a wrist-mounted living body information measuring instrument to be used being attached to a wrist of a measurement-subject person where a radius artery (blood pressure measurement target) exists.
A body (main body) of the blood pressure measuring instrument 200 is equipped with a body unit 101, a body unit 102, and a hinge 103 which connects the body units 101 and 102. The body unit 102 is connected to the body unit 101 by the hinge 103 so as to be swingable with respect to the latter.
The inner circumferential surfaces of the body units 101 and 102 are shaped so as to conform to the outer shape of a wrist H. A space Ka into which the wrist H of the measurement-subject person can be inserted is formed between the body units 101 and 102 in a state that an end portion, opposite to the hinge 103, of the body unit 101 is set closest to the body unit 102.

The body units 101 and 102 are fixed to each other by a belt 106 in a state that the wrist H is inserted in the space Ka, whereby the blood pressure measuring instrument 200 is fixed (attached) to the wrist H.

The body unit 101 is equipped with a pressure pulse wave detection unit 100 which is disposed at such a position as to be opposed to the wrist H in a state that the blood pressure measuring instrument 200 is attached to the wrist H and a display unit 104 which functions as a first display unit mounted in the outer circumferential surface.

The body unit 102 is equipped with a display unit 105 which functions as a second display unit mounted in its outer circumferential surface.

The display units 104 and 105 are disposed such that the space Ka which is formed by the body which is composed of the body units 101 and 102 and the hinge 103 exists on a straight line L that connects the center of the display screen of the display unit 104 and the center of the display screen of the display unit 105. The center of the display screen of the display unit 104 and the center of the display screen of the display unit 105 are different from each other in their positions in a direction D that is perpendicular to the palm of the hand of the measurement-subject person in a state that the blood pressure measuring instrument 200 is attached to the wrist H of the measurement-subject person.

In the example of Fig. 1, in the direction D, the display unit 104 is disposed on the palm side of the center of the space Ka and the display unit 105 is disposed on the hand back side of the center of the space Ka.

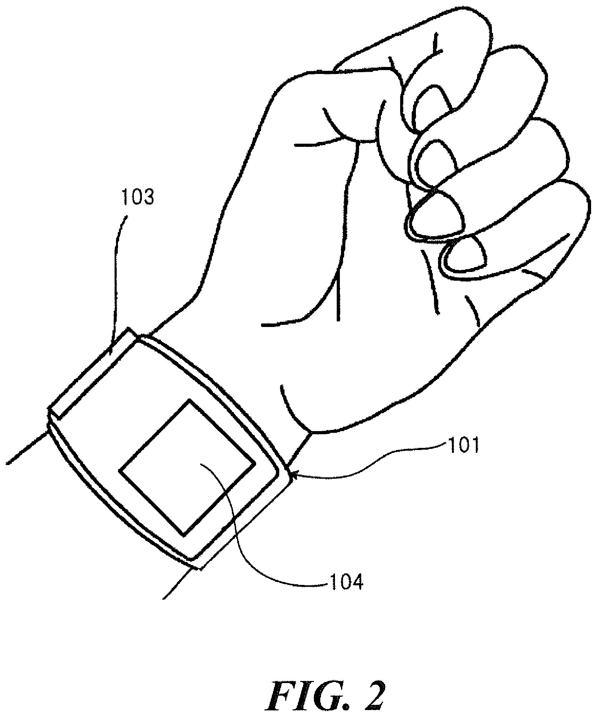

Fig. 2 is a view, as viewed from the palm side, of the blood pressure measuring instrument 200 being attached to the left wrist of the measurement-subject person. As shown in Fig. 2, the outer circumferential surface of the body unit 101 is formed with the display unit 104, which is therefore be viewable from the palm side.

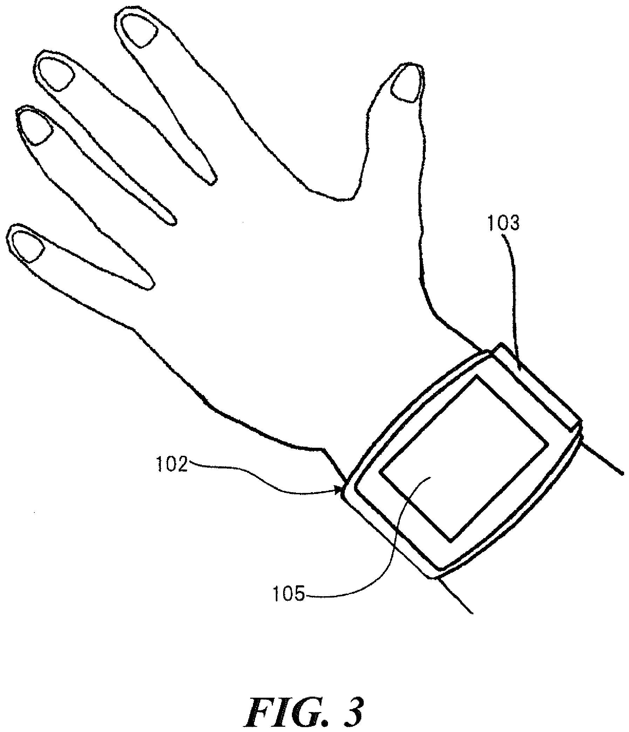

Fig. 3 is a view, as viewed from the hand back side, of the blood pressure measuring instrument 200 being attached to the left wrist of the measurement-subject person. As shown in Fig. 3, the outer circumferential surface of the body unit 102 is formed with the display unit 105, which is therefore viewable from the hand back side.

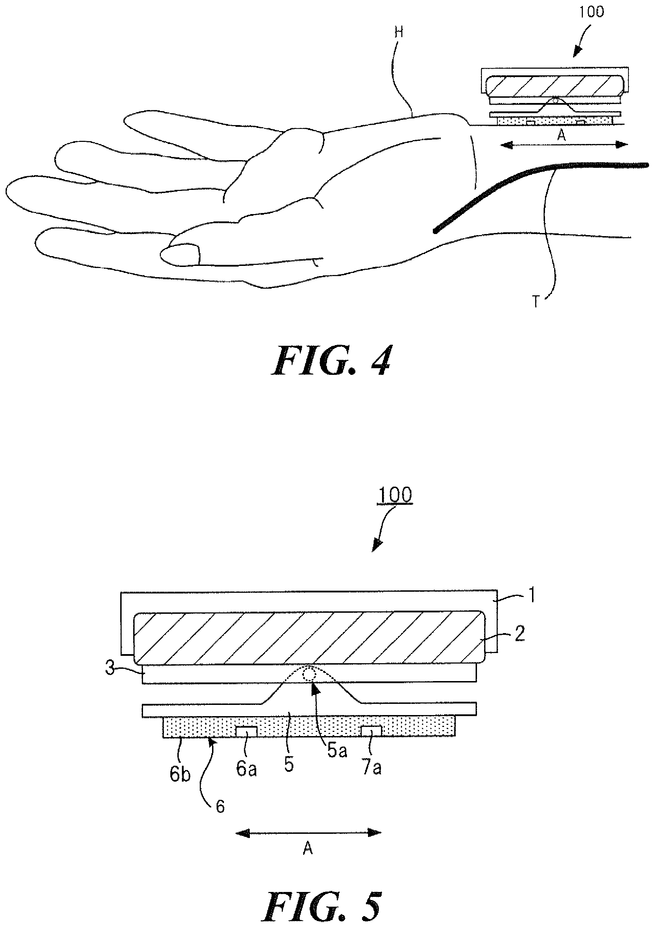

Fig. 4 is an appearance view showing the configuration of the pressure pulse wave detection unit 100 of the blood pressure measuring instrument 200 of Figs. 2 and 3 being attached to the wrist. Only the pressure pulse wave detection unit 100 of the blood pressure measuring instrument 200 is shown in Fig. 4.

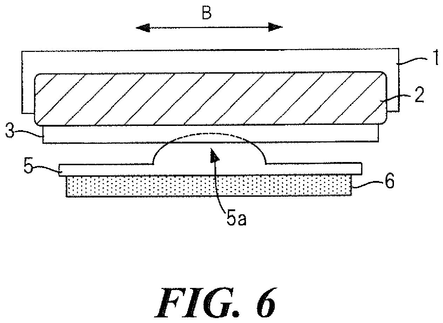

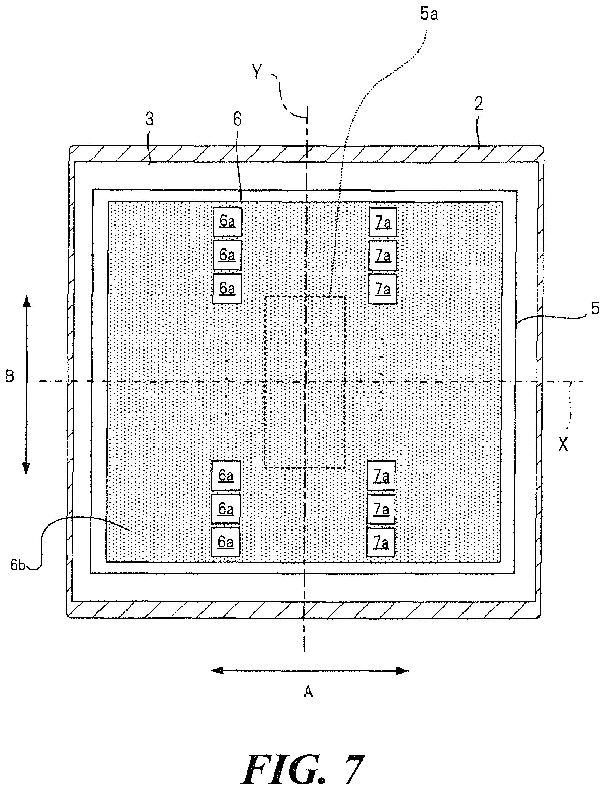

Fig. 5 is an enlarged view of the pressure pulse wave detection unit 100 shown in Fig. 4. Fig. 6 is a view, as viewed from the side of the fingertips of the measurement-subject person, of the pressure pulse wave detection unit 100 shown in Fig. 4 that is in an attached state. Fig. 7 is a view, as viewed from the side of the part being in contact with the wrist, of the pressure pulse wave detection unit 100 shown in Fig. 4 that is in an attached state. Figs. 4 to 7 are schematic views of pressure pulse wave detection unit 100 and should not be construed as restricting the dimensions of individual portions, their arrangement, and other things.

The pressure pulse wave detection unit 100 is equipped with a body 1 which incorporates an air bag 2, a flat plate member 3 which is fixed to the air bag 2, a rotary member 5 which is supported by a biaxial rotating mechanism 5a so as to be rotatable about each of two axes relative to the flat plate member 3, and a sensor unit 6 which is attached to a flat surface, opposite to the flat plate member 3, of the rotary member 5.

The air bag 2 functions as a pressing unit for pressing a pressing surface 6b of the sensor unit 6 against an artery that is located under the skin of a living body part (wrist) in a state that the blood pressure measurement device 200 is attached to the wrist. The pressing unit may be any kind of unit as long as it can press the pressing surface 6b of the sensor unit 6 toward an artery; it is not limited to units that employ an air bag.

The amount of air existing inside the air bag 2 is controlled by a pump (not shown), whereby the air bag 2 moves the flat plate member 3 which is fixed to the air bag 2 in the direction that is perpendicular to a surface (a flat surface located on the rotary member 5 side) of the flat plate member 3.

In the attached state shown in Fig. 4, the pressing surface 6b of the sensor unit 6 which is included in the pressure pulse wave detection unit 100 is in contact with the skin of the wrist of the measurement-subject person. When the amount of air injected in the air bag 2 is increased in this state, the internal pressure of the air bag 2 is increased, whereby the sensor unit 6 is pushed toward a radius artery T that exists in the wrist. The following description will be made with an assumption that the pressure force that is exerted toward the radius artery T by the sensor unit 6 is equivalent to the internal pressure of the air bag 2.

As shown in Fig. 7, the pressing surface 6b is formed with plural pressure sensors 6a (pressure detecting elements) which are arranged in a direction B that crosses (in the example of Fig. 4, is perpendicular to) a direction A in which the radius artery T extends that exists in the attachment target part, in the attached state shown in Fig. 4. The pressing surface 6b is also formed with plural pressure sensors 7a which are arranged in the direction B. Each pressure sensor 6a and a pressure sensor 7a that is located at the same position as the pressure sensor 6a in the direction B constitute a pair, and plural such pairs are arranged in the direction B on the pressing surface 6b. The pressure sensors (plural pressure sensors 6a and plural pressure sensors 7a) included in the pressure pulse wave detection unit 100 constitute a pressure detection unit.

The pressing surface 6b is a surface of a semiconductor substrate which is made of, for example, single crystal silicon and the pressure sensors 6a and 7a are, for example, pressure-sensitive diodes formed on the surface of the semiconductor substrate.

The pressure sensors 6a (7a) are pressed against the radius artery T such that its arrangement direction crosses (is approximately perpendicular to) the radius artery T, and the pressure sensors 6a detect a pressure vibration wave (i.e., pressure pulse wave) that is generated from the radius artery T and transmitted to the skin. The pressure pulse wave is included in living body information.

The interval between the pressure sensors 6a (7a) is set sufficiently small so that a necessary and sufficient number of pressure sensors 6a (7a) are arranged over the radius artery T. The arrangement length of the pressure sensors 6a (7a) is set necessarily and sufficiently greater than the diameter of the radius artery T.

As shown in Fig. 7, the biaxial rotating mechanism 5a is a mechanism for rotating the rotary member 5 about each of two rotation axes X and Y which are perpendicular to the direction in which the flat plate member 3 is pushed by the air bag 2.

The biaxial rotating mechanism 5a has the two orthogonal rotation axes X and Y which are set on the surface of the flat plate member 3 and about each of which rotational driving is performed by a rotational drive unit 10 (described later).

The rotation axis Y is a first axis that extends in the arrangement direction of the plural pressure sensors 6a (7a) formed on the pressing surface 6b. As shown in Fig. 7 (plan view), the rotation axis Y is set between (in the example of Fig. 7, at the center of) the element array of the plural pressure sensors 6a and the element array of the plural pressure sensors 7a.

The rotation axis X is a second axis that extends in the direction that is perpendicular to the arrangement direction of the plural pressure sensors 6a (7a) formed on the pressing surface 6b. In the example of Fig. 7, the rotation axis X is set as a straight line that equally divides each of the element array of the plural pressure sensors 6a and the element array of the plural pressure sensors 7a.

When the rotary member 5 is rotated about the rotation axis X, the pressing surface 6b is rotated about the rotation axis X. When the rotary member 5 is rotated about the rotation axis Y, the pressing surface 6b is rotated about the rotation axis Y.

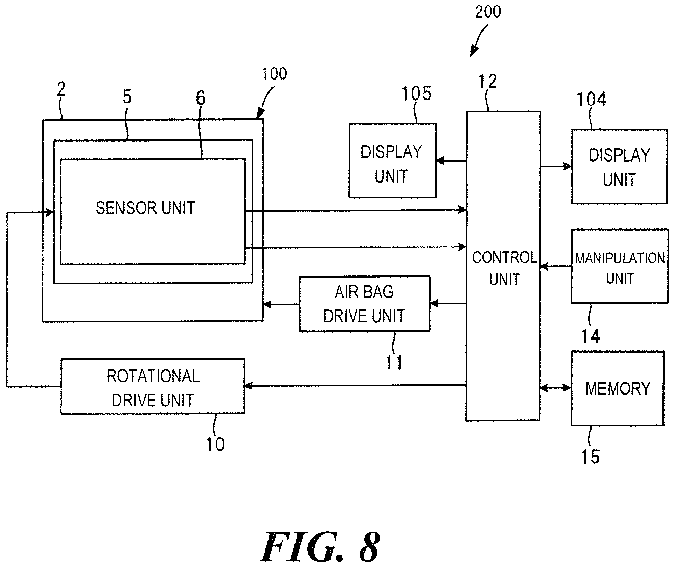

Fig. 8 shows a block configuration of the blood pressure measuring instrument 200.

The blood pressure measuring instrument 200 is equipped with the pressure pulse wave detection unit 100, a rotational drive unit 10, an air bag drive unit 11, a control unit 12 for centralized control of the entire instrument, the display units 104 and 105, a manipulation unit 14, and a memory 15.

The rotational drive unit 10 is an actuator that is connected to each of the rotation axes (shafts) X and Y of the biaxial rotating mechanism 5a of the pressure pulse wave detection unit 100. The rotational drive unit 10 rotates the pressing surface 6b about the rotation axes X and Y by rotationally driving the rotation axes (shafts) X and Y individually according to an instruction from the control unit 12.

The air bag drive unit 11 controls the amount of air to be injected in the air bag 2 (an internal pressure of the air bag 2) according to an instruction from the control unit 12.

Each of the display units 104 and 105 serves to display various kinds of information such as measured blood pressure values and employs, for example, a liquid crystal display device, an organic electroluminescence display device, or an electronic paper display.

The manipulation unit 14, which is an interface for input of an instruction signal for the control unit 12, is composed of buttons for commanding a start of any of various operations including a blood pressure measurement and other components.

The memory 15 includes a ROM (read-only memory) for storing programs and data for allowing the control unit 12 to perform prescribed operations, a RAM (random access memory) as a working memory, a flash memory for storing various kinds of information such as measured blood pressure data, and other components.

The control unit 12 functions as a pressing control unit, a living body information measuring unit, a rotation control unit, a calibration data generation unit, and a display control unit by running the programs stored in the ROM of the memory 15.

The pressing control unit controls the pressing force that the pressing surface 6b exerts on the wrist by adjusting the amount of air that occupies the inside the air bag 2 by controlling the air bag drive unit 11.

The living body information measuring unit calculates first blood pressure values in the radius artery T on the basis of pressure pulse waves that are detected by the pressure sensors 6a and 7a formed in the pressing surface 6b in a state that the pressing surface 6b is pressed toward the radius artery T.

More specifically, the living body information measuring unit calculates first blood pressure values in the radius artery T on the basis of pressure pulse waves that were detected by the pressure sensors 6a and 7a in a process that the pressing force acting on the radius artery T was varied (increased or decreased) by the air bag drive unit 11.

The calibration data generation unit generates calibration data using the first blood pressure values calculated by the first blood pressure calculation unit.

The rotation control unit judges whether it is necessary for the rotational drive unit 10 to rotate the pressing surface 6b, on the basis of pressure pulse waves that were detected by the pressure sensors 6a and 7a in a process that the pressing force acting on the radius artery T was increased by the air bag drive unit 11. If judging that rotation is necessary, the rotation control unit causes the rotational drive unit 10 to rotate the pressing surface 6b.

The living body information measuring unit calculates second blood pressure values in the radius artery T for each beat by calibrating, using the calibration data, a pressure pulse wave that is detected by the pressure sensors 6a and 7a for each beat in a state that the pressing surface 6b is pressed toward the radius artery T with an optimum pressing force for deforming part of the radius artery T into a flat shape. Furthermore, the living body information measuring unit calculates a pulse rate on the basis of pressure pulse waves detected for respective beats.

The display control unit performs a display control on each of the display units 104 and 105.

A description will be made below of how the blood pressure measuring instrument according to the embodiment operates. The blood pressure measuring instrument according to the embodiment has a continuous blood pressure measurement mode in which a blood pressure value SBP (systolic blood pressure; what is called a maximum blood pressure) and a blood pressure value DBP (diastolic blood pressure; what is called a minimum blood pressure) are measured and displayed on the display unit 104 beat by beat.

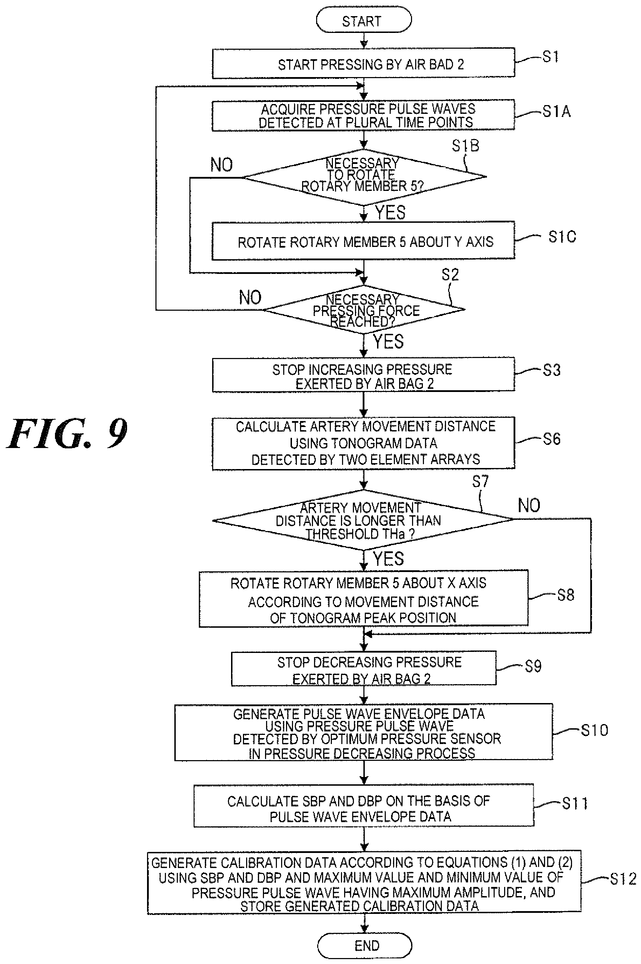

Fig. 9 is a flowchart for illustrating an operation, to generation of calibration data in the continuous blood pressure measurement mode, of the pressure measurement device 200.

It is assumed that in an initial state, that is, a state before reception of a blood pressure measurement instruction, the rotation amount of the rotary member 5 of the pressure pulse wave detection unit 100 is set at, for example, zero, that is, the pressing surface 6b is parallel with the flat plate member 3.

Although the state that the rotation amount is set at zero is employed here as the initial state, the invention is not limited this case. For example, the initial state may be a state that the rotational drive unit 10 has rotated the pressing surface 6b so that the pressing surface 6b comes into uniform contact with the skin according to the shape of the wrist to which the blood pressure measurement device is attached.

Upon reception of a blood pressure measurement instruction, the control unit 12 controls the air bag drive unit 11 to start injecting air into the air bag 2 and thereby increases the pressing force that the pressing surface 6b exerts toward the radius artery T (step S1).

In the process of increasing the pressing force, with certain timing (e.g., periodic timing) after a lapse of a sufficient time from a start of closure of the radius artery T, the control unit 12 acquires plural latest pressure pulse wave information I1 (pressure pulse waves) that have been detected by the respective pressure sensors 6a so far and are stored in the memory 15. With the certain timing, the control unit 12 acquires plural latest pressure pulse wave information I2 (pressure pulse waves) that have been detected by the respective pressure sensors 7a so far and are stored in the memory 15 (step S1A).

The control unit 12 calculates, for example, an amplitude average value Ave1 of pressure pulse waves that were detected by the respective pressure sensors 6a at time t1 among the plural pressure pulse wave information I1 acquired at step 1A and an amplitude average value Ave2 of pressure pulse waves that were detected by the respective pressure sensors 6a at time t2 that is later than time t1. The control unit 12 also calculates an amplitude average value Ave3 of pressure pulse waves that were detected by the respective pressure sensors 7a at time t1 among the plural pressure pulse wave information I2 acquired at step 1A and an amplitude average value Ave4 of pressure pulse waves that were detected by the respective pressure sensors 7a at time t2. Then the control unit 12 calculates ratios between the average values calculated at the same time points, that is, Ave1/Ave3 and Ave2/Ave4.

The control unit 12 judges whether to cause the rotational drive unit 10 to rotate the rotary member 5 on the basis of a variation between the ratios calculated the plural time points. That is, the control unit 12 judges whether to rotate the rotary member 5 on the basis of the pressure pulse waves detected by the pressure sensors 6a and 7a at the plural time points in the process that the pressing force is increased (step SIB).

For example, if the ratios calculated at the plural time points show monotonic increase, it can be judged that the element array of the pressure sensors 7a are located on the side on which the radius artery T is closed and the element array of the pressure sensors 6a are not located on that side. Thus, the control unit 12 judges that it is necessary to rotate the rotary member 5.

If the ratios calculated at the plural time points show monotonic decrease, it can be judged that the element array of the pressure sensors 6a are located on the side on which the radius artery T is closed and the element array of the pressure sensors 7a are not located on that side. Thus, the control unit 12 judges that it is necessary to rotate the rotary member 5.

If the ratios calculated at the plural time points show almost no variation, it can be judged that the two element arrays are detecting pressure pulse waves of the radius artery T in the same manner. Thus, the control unit 12 judges that it is not necessary to rotate the rotary member 5.

If the ratios calculated at plural time points show increase and decrease repeatedly, it cannot be judged whether the radius artery T is pressed sufficiently on the respective sides of the two element arrays or the radius artery T is not pressed sufficiently only on the side of one element array. Thus, the control unit 12 judges that it is not necessary to rotate the rotary member 5.

As described above, the control unit 12 judges whether rotation is necessary on the basis of a variation of the ratios calculated at the plural time points. Instead of the ratios, a difference (a sign is taken into consideration) between the average values Ave1 (Ave2) and Ave3 (Ave4) may be used.

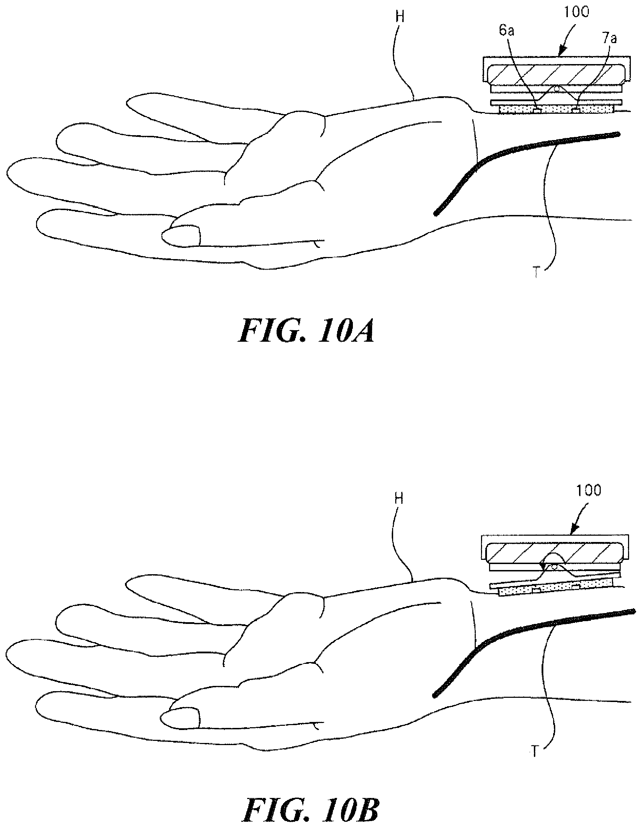

Fig. 10A is a diagram for showing an example state that the radius artery T is closed on the side of the element array of the pressure sensors 7a but is not closed on the side of the element array of the pressure sensors 6a. In the state of Fig. 10A, the distance between the element array of the pressure sensors 6a is longer than that between the element array of the pressure sensors 7a.

Let 6A and 7A represent an amplitude average value of pressure pulse waves detected by the respective pressure sensors 6a and an amplitude average value of pressure pulse waves detected by the respective pressure sensors 7a, respectively; then, in the state of Fig. 10, the ratio 6A/7A is sufficiently larger than 1. In this state, 6A/7A comes closer to 1 if the element array of the pressure sensors 6a is brought closer to the radius artery T.

Thus, if judging at step S1B that it is necessary to rotate the rotary member 5 about the rotation axis Y, the control unit 12 performs a control so as to rotate the rotary member 5 about the rotation axis Y according to a 6A/7A value obtained at the latest time point (step S1C).

More specifically, the control unit 12 reads out a rotation amount corresponding to the 6A/7A value by referring to a data table (determined empirically and stored in the memory 15 before shipment of a product) showing a relationship between the 6A/7A value and the rotation amount of the rotary member 5 and sets the read-out rotation amount.

In addition, the control unit 12 judges which of the average value 6A and the average value 7A is larger. If the average value 6A is larger, the control unit 12 sets the rotation direction of the rotary member 5 about the rotation axis Y to counterclockwise in Fig. 10 to decrease the distance between the element array of the pressure sensors 6a and the radius artery T.

If the average value 7A is larger, the control unit 12 sets the rotation direction of the rotary member 5 about the rotation axis Y to clockwise in Fig. 10 to decrease the distance between the element array of the pressure sensors 7a and the radius artery T.

The control unit 12 rotates the rotary member 5 in the thus-set rotation direction by the thus-set rotation amount. As a result, as shown in Fig. 10B, the pressing surface 6b can be made parallel with the radius artery T to establish a state that the radius artery T is closed on the respective sides of the two element arrays.

The control unit 12 moves to step S2 after the execution of step S1C or after the execution of step S1B (if a judgment was made that it is not necessary to rotate the rotary member 5). At step S2, the control unit 12 judges whether the pressing force has become such as to correspond to a pressure that is enough to close the radius artery T (i.e., has reached a necessary pressing force). If judging that the pressing force has reached a necessary pressing force (step S2: yes), the control unit 12 controls the air bag drive unit 11 to stop the injection of air into the air bag 2 (step S3). If judging that the pressing force has not reached a necessary pressing force yet, the control unit 12 returns to step S1A.

After the execution of step S3, the control unit 12 determines an amplitude distribution curve (what is called a tomogram) indicating a relationship between the amplitude of a pressure pulse wave that was detected by each pressure sensor 6a at each time point from step S1 to step S3 and the position of the pressure sensor 6a in the pressing surface 6b. The control unit 12 also determines a tomogram indicating a relationship between the amplitude of a pressure pulse wave that was detected by each pressure sensor 7a at each time point and the position of the pressure sensor 7a in the pressing surface 6b.

The control unit 12 stores the tomogram generated for the element array of the pressure sensors 6a in the memory 15 such that it is correlated with identification information of the element array, a detection time of the pressure pulse waves, and a pressing force of the air bag 2 in the pressing direction (an internal pressure of the air bag 2) at the detection time.

Likewise, the control unit 12 stores the tomogram generated for the element array of the pressure sensors 7a in the memory 15 such that it is correlated with identification information of the element array, a detection time of the pressure pulse waves, and a pressing force of the air bag 2 in the pressing direction at the detection time.

The control unit 12 calculates a movement distance of the radius artery T in the direction B during the pressing of the pressing surface 6b against the wrist using the tomogram data stored in the memory 15 (step S6).

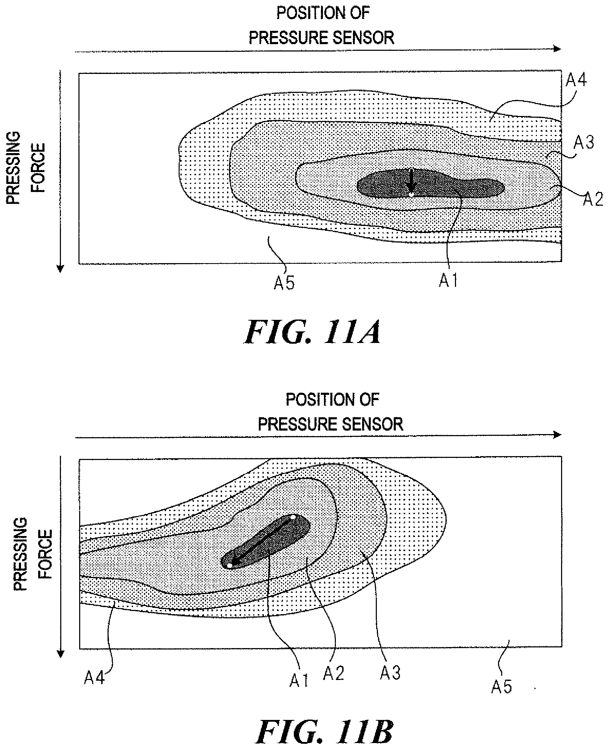

Figs. 11A and 11B are diagrams for showing examples of amplitude values of pressure pulse waves that are detected by the pressure sensors 6a of the sensor unit 6 as the pressing force that the sensor unit 6 exerts on the wrist is varied. In Figs. 11A and 11B, the horizontal axis represents the position of the pressure sensor 6a in the direction B and the vertical axis represents the pressing force.

In Figs. 11A and 11B, the amplitudes of the pressure pulse waves detected by the pressure sensors 6a located at the respective positions are indicated by different colors according to their magnitudes.

Symbol A1 denotes a region where the amplitude is larger than or equal to a threshold TH1. Symbol A2 denotes a region where the amplitude is larger than or equal to a threshold TH2 and smaller than the threshold TH1. Symbol A3 denotes a region where the amplitude is larger than or equal to a threshold TH3 and smaller than the threshold TH2. Symbol A4 denotes a region where the amplitude is larger than or equal to a threshold TH4 and smaller than the threshold TH3. Symbol A5 denotes a region where the amplitude is smaller than the threshold TH4. There is a relationship that (threshold TH1) > (threshold TH2) > (threshold TH3) > (threshold TH4).

The example of Fig. 11A is such that the positions of pressure sensors 6a that detect pressure pulse waves whose amplitudes are larger than or equal to the threshold TH1 have almost no changes in a process that the pressing force is increased. In contrast, the example of Fig. 11B is such that the positions of pressure sensors 6a that detect pressure pulse waves whose amplitudes are larger than or equal to the threshold TH1 shift leftward in a process that the pressing force is increased.

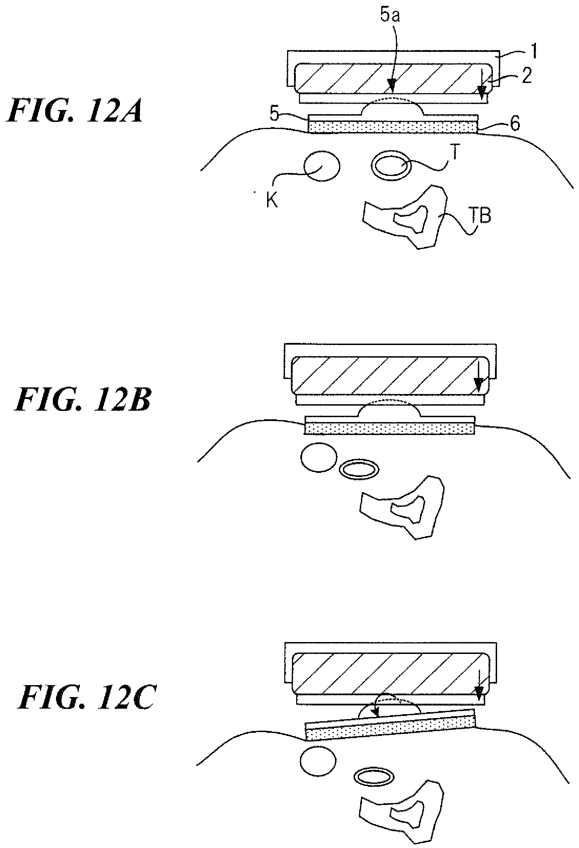

Figs. 12A to 12C are diagrams for showing how pressing of the sensor unit 6 against the wrist by the air bag 2 proceeds after attachment of the pressure pulse wave detection unit 100 to the wrist. In Figs. 12A to 12C, symbol TB denotes the radius and symbol K denotes a tendon.

After pressing of the sensor unit 6 against the wrist by the air bag 2 is started as shown in Fig. 12A, there may occur an event that the radius artery T is moved in the direction B as shown in Fig. 12B.

If the radius artery T is moved in the direction B as shown in Fig. 12B during pressing, the distribution of the amplitude values of pressure pulse waves vary as shown in Fig. 11B during the pressing. More specifically, there occurs a large difference between the position of a pressure sensor 6a that detects an amplitude value that is larger than or equal to the threshold TH1 first as the pressing force is increased and the position of a pressure sensor 6a that detects an amplitude value that is larger than or equal to the threshold TH1 last.

In the example of Fig. 11A, there is no large difference between the position of a pressure sensor 6a that detects an amplitude value that is larger than or equal to the threshold TH1 first as the pressing force is increased and the position of a pressure sensor 6a that detects an amplitude value that is larger than or equal to the threshold TH1 last. Thus, it is seen that the radius artery T is closed making almost no movement in the direction B in the process that the pressing force is increased.

In this manner, a position variation of the radius artery T in the direction B can be detected by checking how the tomogram varies in a process that the pressing force is changed. If the radius artery T is closed by increasing the pressing force while the state of Fig. 12B is left as it is, correct tonograms may not be obtained due to influence from living body tissue such as the tendon K.

In view of the above, at step S6, the control unit 12 calculates a difference (i.e., a movement distance of the radius artery T in the direction B) between the position of a pressure sensor 6a that detected an amplitude value that is larger than or equal to the threshold TH1 first as the pressing force was increased and the position of a pressure sensor 6a that detected an amplitude value that is larger than or equal to the threshold TH1 last on the basis of data as shown in Figs. 11A and 11B that indicate a relationship between the pressing force and the tomogram. And the control unit 12 judges whether the calculated difference is larger than or equal to a threshold THa (step S7).

If the difference between the two positions is larger than or equal to the threshold THa (step S7: yes), at step S8 the control unit 12 determines a vector as shown in Fig. 11B by an arrow. If the difference between the two positions is smaller than the threshold THa (step S7: no), the control unit 12 moves to step S9.

Information indicating a relationship between the direction and magnitude of a vector as shown in Figs. 11A and 11B and the direction and amount of rotation of the rotary member 5 about the rotation axis X is determined empirically and stored in the memory 15 in advance.

The control unit 12 acquires information indicating a rotation direction and amount corresponding to the determined direction and magnitude of the determined vector from the memory 15, and sends the acquired information to the rotational drive unit 10. The rotational drive unit 10 rotates the rotary member 5 according to the received information in the manner shown in Fig. 12C (step S8).

As described above, when receiving a blood pressure measurement instruction, the control unit 12 judges whether it is necessary to rotate the rotary member 5 at steps S1B and S7 on the basis of pressure pulse waves detected by the respective pressure sensors 6a and 7a at plural time points in the process that the pressing force of the air bag 2 was increased. If judging that it is necessary to rotate the rotary member 5 (step S1B: yes; step S7: yes), the rotational drive unit 10 rotates the rotary member 5 on the basis of the pressure pulse waves detected by the respective pressure sensors 6a and 7a.

At step S9 which follows step S8, the control unit 12 starts decreasing the pressing force acting on the radius artery T by discharging air from the air bag 2.

Upon decreasing the pressing force to a minimum value after the reduction of the pressing force was started at step S9, the control unit 12 determines an optimum pressure sensor from among all of the pressure sensors 6a and 7a. For example, the control unit 12 determines, as an optimum pressure sensor, a pressure sensor that detected a pressure pulse wave having a maximum amplitude in the process that the pressing force was decreased.

A pressure pulse wave that is detected by a pressure sensor that is located right over a flat portion of the radius artery T is not affected by tension of the wall of the radius artery T and hence exhibits a maximum amplitude. And this pressure pulse wave provides a highest correlation with the blood pressure inside the radius artery T. For these reasons, a pressure sensor that detected a pressure pulse wave having a maximum amplitude is determined as an optimum pressure sensor.

Plural pressure sensors may be found that detected pressure pulse waves having a maximum amplitude. In this case, it is appropriate to deal with these plural pressure sensors as optimum pressure sensors and to employ an average, for example, of the pressure pulse waves detected by these respective plural pressure sensors as a "pressure pulse wave detected by an optimum pressure sensor."

The control unit 12 generates pulse wave envelope data using the pressure pulse wave detected by the optimum pressure sensor in the pressing force decreasing process (step S10).

The pulse wave envelope data is data that correlates the pressing force (the internal pressure of the air bad 2) that the sensor unit 6 exerts toward the radius artery T and the amplitude of the pressure pulse wave that is detected by the optimum pressure sensor in a state that the optimum pressure sensor is pressed toward the radius artery T by this pressing force.

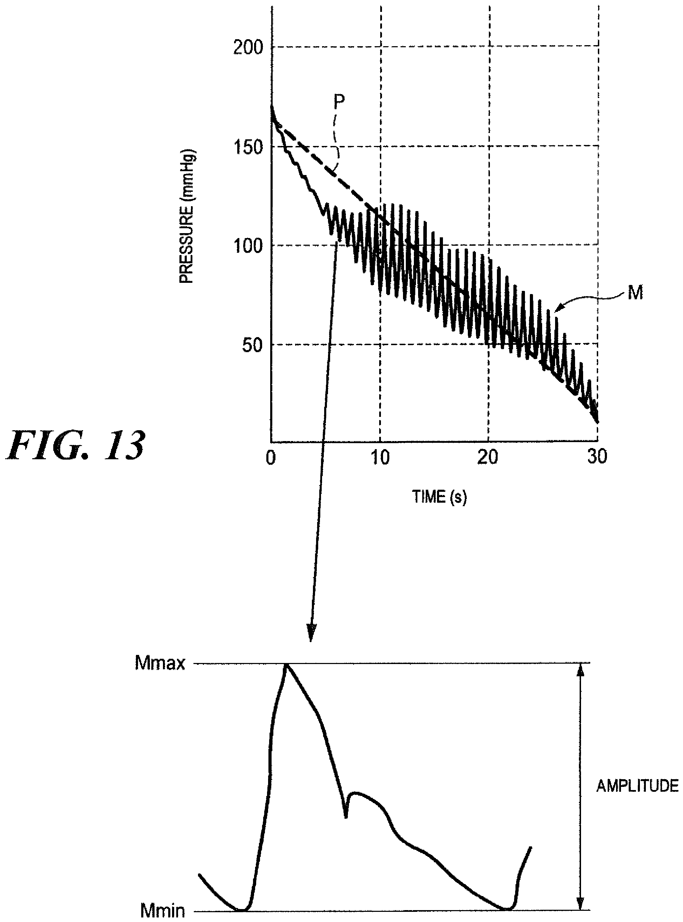

Fig. 13 is a graph for showing an example of how a pressure pulse wave detected by an optimum pressure sensor varies as the pressure acting on the wrist is varied. In Fig. 13, a straight line that is given a symbol P indicates the pressure and a waveform that is given a symbol M denotes a pressure wave. An enlarged version of a one-beat pressure pulse wave is shown in a bottom part of Fig. 13.

As shown in Fig. 13, pressures at a rising point and a falling point of each pressure pulse wave are referred to as a minimum value Mmin and a maximum value Mmax, respectively. The amplitude of the pressure pulse wave is a difference between the maximum value Mmax and the minimum value Mmin. Each of the maximum value Mmax and the minimum value Mmin is information characterizing the shape of the pressure pulse wave.

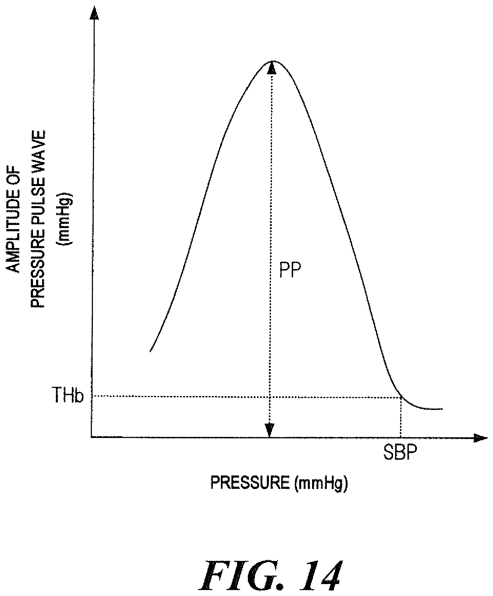

Upon cancellation of a closed state of the radius artery T after a start of reduction of the pressing force, the amplitude of the pressure pulse wave detected by the optimum pressure sensor increases rapidly. The amplitude thereafter varies as shown in Fig. 13 as the pressing force decreases. At step S10, the control unit 12 generates pulse wave envelope data as shown in Fig. 14 on the basis of the relationship between the pressing force and the pressure pulse wave as shown in Fig. 13.

Upon generating the pulse wave envelope data as shown in Fig. 14, the control unit 12 calculates SBP and DBP on the basis of the generated pulse wave envelope data (step S11).

For example, the control unit 12 determines, as SBP, a pressure at a time point when the pressure pulse wave amplitude starts to rise rapidly in the pulse wave envelope shown in Fig. 14 after the start of reduction of the pressing force, that is, a pressure at a time point when the pressure pulse wave amplitude detected by the optimum pressure sensor first exceeds a threshold THb for judgment for an end of an artery closed state after the start of reduction of the pressing force. Alternatively, the control unit 12 calculates a difference between two adjacent amplitude values of the pulse wave envelope data and determines, as SBP, a pressure at a time point when the difference exceeds a threshold.

Furthermore, the control unit 12 employs, as a pulse pressure (PP), a maximum value of the pressure pulse wave amplitude of the pulse wave envelope shown in Fig. 14 and calculates DBP using the calculated SBP and PP according to an equation SBP - DBP = PP.

After the execution of step S11, the control unit 12 generates calibration data to be used in a continuous blood pressure measurement (described later) using a maximum value Mmax and a minimum value Mmin of one (e.g., a pressure pulse wave that had a maximum amplitude) of pressure pulse waves detected by the optimum pressure sensor that was determined in the pressure decreasing process (step S9) and the SBP and DBP calculated at step S11. The control unit 12 stores the generated calibration data in the memory 15 (step S12).

Relationships

$$ SBP = a \times Mmax + b \qquad \cdots (1) $$

$$ DBP = a \times Mmin + b \qquad \cdots (2) $$

holds where **a** and **b** are a slope and an intercept of a linear function, respectively.

The control unit 12 calculates the slope **a** and the intercept **b** by substituting the SBP and DBP determined at step S11 and the maximum value Mmax and the minimum value Mmin of the pressure pulse wave having a maximum amplitude in the pulse wave envelope shown in Fig. 14 into Equations (1) and (2). The control unit 12 stores the calculated coefficients **a** and **b** and Equations (1) and (2) in the memory 15 as calibration data.

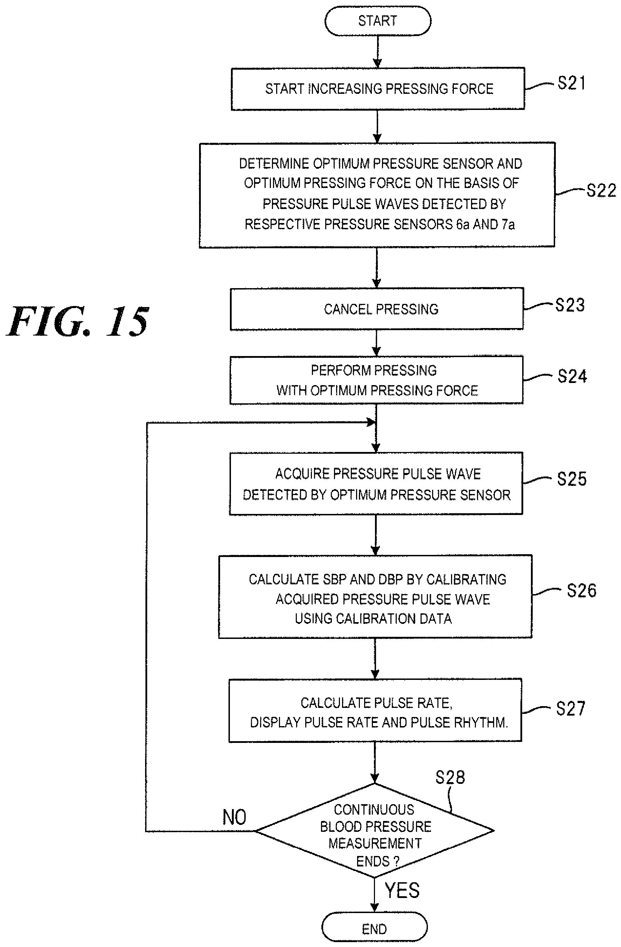

Fig. 15 is a flowchart illustrating a continuous blood pressure measuring operation in a continuous blood pressure measurement mode of the blood pressure measuring instrument according to the embodiment.

After generating calibration data according to the flowchart shown in Fig. 9, the control unit 12 controls the air bag drive unit 11 to increase the internal pressure of the air bag 2 and thereby increase the pressing force that the pressing surface 6b exerts toward the radius artery T (step S21).

Then the control unit 12 determines, as an optimum pressure sensor, one, that detected a pressure pulse wave having a maximum amplitude, of the pressure sensors 6a and 7a in the process that the pressing force was increased. And the control unit 12 determines, as a pressure corresponding to an optimum pressing force, an internal pressure of the air bag 2 that was produced at a time point of detection of the pressure pulse wave having the maximum amplitude (step S22).

Then the control unit 12 restores the initial state by releasing the air from inside the air bag 2 (step S23). The control unit 12 thereafter increases the internal pressure of the air bag 2 to the pressure corresponding to the optimum pressing force determined at step S22, and maintains the optimum pressing force (step S24).

Subsequently, in a state that the pressing surface 6b is pressed toward the radius artery T with the optimum pressing force, at step S25 the control unit 12 acquires a pressure pulse wave that is detected by the optimum pressure sensor determined at step S22.

Then, the control unit 12 calculates SBP and DBP by calibrating the acquired one-beat pressure pulse wave using the calibration data that was generated at step S12 in Fig. 9, and causes the display unit 104 to display the calculated SBP and DBP (step S26).

More specifically, the control unit 12 calculates SBP by substituting a maximum value Mmax of the pressure pulse wave acquired at step S25 and the coefficients **a** and **b** calculated at step S12 into the above-mentioned Equation (1) and calculates DBP by substituting a minimum pressure value Mmin of the pressure pulse wave acquired at step S25 and coefficients **a** and **b** calculated at step S12 into the above-mentioned Equation (2).

The control unit 12 stores the pressure pulse wave acquired at step S25 in the memory such that it is correlated with a time of its acquisition, and calculates, as a pulse rate, the number of pressure pulse waves (stored in the past) per prescribed time (e.g., 1 min). The control unit 12 causes the display 105 to display pulse rhythm corresponding to the calculated pulse rate in the form of information other than a numeral, and causes the display 104 to display the calculated pulse rate (step S27).

After the execution of step S27, the control unit 12 finishes the process if receiving an instruction to finish the continuous blood pressure measurement (step S28: yes), and returns to step S25 if not receiving an end instruction (step S28: no).

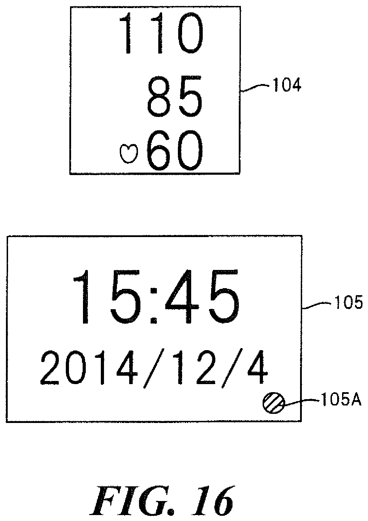

Fig. 16 shows example displays to be made on the screens of the display units 104 and 105.

As shown in Fig. 16, the SBP (in the illustrated example, "110") and the DBP (in the illustrated example, "85") that were calculated at step S26 for each beat and the pulse rate (in the illustrated example, "60") calculated at step S27 are displayed on the display unit 104.

An icon 105A is displayed on the display unit 105 so as to blink according to the pulse rhythm of the pulse rate calculated at step S27. If the pulse rate calculated at step S27 is "60," the icon 105A blinks 60 times per minute. The control unit 12 causes the display unit 105 to also display information (in the example of Fig. 16, date-and-time information) that does not relate to any living body information.

As described above, in the blood pressure measuring instrument 200, in the continuous blood pressure measurement mode, blood pressure values and a pulse rate that are calculated for each beat are displayed only on the display unit 104 and are not displayed on the display unit 105. Since the display unit 104 is disposed at such a position as to be viewable from the side of the palm of the measurement-subject person, it is hard to see to persons other than the measurement-subject person. This measure thus enables preservation of privacy.

On the other hand, in the blood pressure measuring instrument 200, in the continuous blood pressure measurement mode, the icon 105A is displayed on the display unit 105 so as to blink according to a pulse rate that is measured at the same time as blood pressure values. Since the display screen of the display unit 105 is located at such a position as to be viewable from the hand back side, the measurement-subject person can see the display unit 105 readily. Furthermore, by looking at the icon 105A being displayed on the display unit 105, the measurement-subject person can recognize whether his or her heartbeat is fast or slow. If pressure pulse waves have not been detected, a pulse rate is not calculated and the icon 105A is not displayed. Thus, by checking the icon 105A, the measurement-subject person can recognize whether a blood pressure measurement is being made correctly.

Persons other than the owner of the blood pressure measuring instrument 200 cannot understand for what purpose the icon 105A is being displayed, that is, blinking. Thus, even if a third person sees the icon 105A, the privacy of the measurement-subject person is still preserved.

The blood pressure measuring instrument 200 can be provided with a mode in which to measure a blood pressure with desired timing and present it to a user. If this mode is set, the blood pressure measuring instrument 200 can measure a blood pressure in a short time and present it to the user without causing him or her to feel troublesome by the control unit 12's executing steps S1-S11 shown in Fig. 9.

It is expected that in this mode a blood pressure measurement is performed in such a situation that results are not seen by others. Thus, blood pressure values and a pulse rate may be displayed on either the display unit 105 or the display unit 104. It is therefore preferable for the control unit 12 to select according to a measurement mode and perform a first control for displaying blood pressure values and a pulse rate only on the display unit 104 or a second control for displaying blood pressure values and a pulse rate on both display units 104 and 105. For example, the control unit 12 may perform the second control in the above-described mode and perform the first control in the continuous blood pressure measurement mode.

Where the blood pressure measuring instrument 200 is attached to an inpatient, it is expected that a health care professional measures blood pressure values and a pulse rate with desired timing. In this case, the blood pressure measuring instrument 200 is not high in usability if blood pressure values and a pulse rate are displayed only on the display unit 104. In view of this, in the case where a health care professional wants to measure blood pressure values and a pulse rate with desired timing, he or she performs a particular manipulation through the manipulation unit 14 and, in response, the control unit 12 performs the second control.

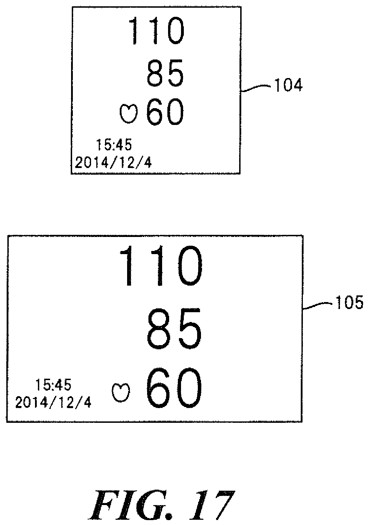

Fig. 17 shows example pictures to be displayed on the display units 104 and 105 by the second control. In the examples shown in Fig. 17, pieces of information, that is, blood pressure values and a pulse rate measured and a date and time are displayed on each of the display units 104 and 105.

When the second control is performed, it becomes possible to see the same information from both of the palm side and the hand back side of the inpatient. As a result, the health care professional can easily check living body information of the inpatient without the need for changing the posture of the wrist of the inpatient. Furthermore, the inpatient and the health care professional can share the same information easily. As a result, it is expected that the health care professional can, for example, make a conversation with the inpatient about his or her physical condition as part of care using the shared information, which could enhance the quality of medical care.

Although the above description is directed to the case that the control unit 12 selects the second control when a blood pressure measurement is performed with desired timing, a configuration is possible in which switching between the first control (i.e., the control for displaying pictures as shown in Fig. 16) and the second control (i.e., the control for displaying pictures as shown in Fig. 17) can be made by a manual manipulation also in the continuous blood pressure measurement mode.

As described above, by virtue of the presence of the two display units 104 and 105, the blood pressure measuring instrument 200 can increase the degree of freedom of selection of a place(s) where to display blood pressure values and a pulse rate as living body information. This provides advantages enhancing the quality of medical care and lowering the burdens of health care professionals and an inpatient while preserving the privacy of the inpatient.

Where the first control is performed, information other than living body information can be displayed on the display unit 105. By causing the display unit 105 to display such an image as an analog watch, this makes it possible to wear the blood pressure measuring instrument 200 in a fashion-oriented manner. This makes it easier to make blood pressure measurement a habit.

The blood pressure measuring instrument 200 may be such that it is also equipped with a living body information measuring device other than the pressure pulse wave detection unit 100, such as a device for measuring a body temperature, a device for measuring a sweat rate, or a device for measuring a percutaneous arterial blood oxygen saturation ($SPO_2$), and that the control unit 12 measures living body information other than blood pressure values and a pulse rate on the basis of information obtained by that device.

In this case, it is appropriate that the first control be such as to display living body information that is not desired to be known to others (blood pressure values, pulse rate, body temperature, sweat rate, or oxygen saturation) only on the display unit 104. It is appropriate that the second control be such as to display blood pressure values, a pulse rate, a body temperature, a sweat rate, or an oxygen saturation on each of the display units 104 and 105.

The control unit 12 may acquire information other than blood pressure values and a pulse rate from an outside device through a wireless or wired communication and display it on the display units 104 and 105. In these modifications, the control unit 12 functions as a living body information acquiring unit for acquiring information other than living body information that is measured on the basis of detection information of the pressure pulse wave detection unit 100.

Whereas the blood pressure measuring instrument 200 shown in Fig. 1 is configured such that the display units 104 and 105 are provided independently of each other, the display units 104 and 105 may be integrated with each other.

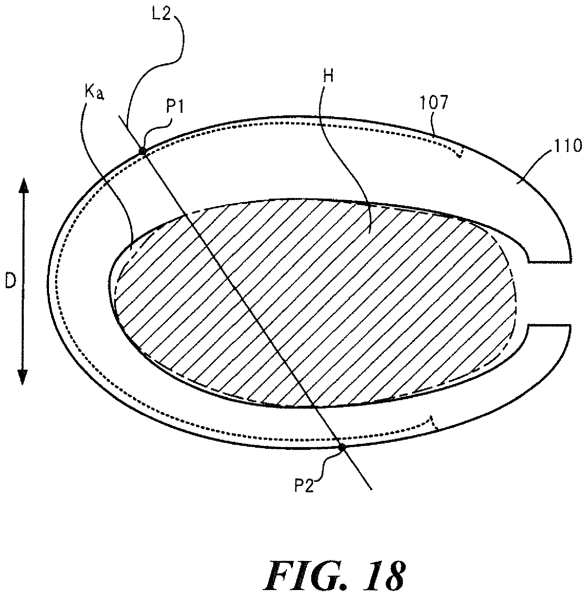

Fig. 18 is a side view showing a modification of the blood pressure measuring instrument 200. The body of the blood pressure measuring instrument is a C-shaped body 110 which is made of a material that enables deformation into a wrist-mountable shape. The body 110 is configured so as to be able to form a space Ka into which a wrist H can be inserted.

The outer circumferential surface of the body 110 is formed with a curved display 107 which extends from the palm side to the hand back side in a state that the blood pressure measuring instrument is attached to the wrist.

In the curved display 107, an area having a prescribed size and centered at point PI that is closer to the palm than the center of the space Ka is in the direction D between two points P1 and P2 where a straight line (e.g., a straight line L2 shown in Fig. 18) passing through the space Ka intersects the outer circumferential surface in Fig. 18 functions as the display unit 104. And an area having a prescribed size and centered at point P2 that is closer to the back of the hand than the center of the space Ka is in the direction D functions as the display unit 105. The configuration shown in Fig. 18 can also provide the same advantages as the blood pressure measuring instrument 200 does.

The processes shown in Figs. 9 and 15 which are executed by the control unit 12 can be implemented as programs for causing a computer to execute their individual steps. Such programs are recorded in a computer-readable, non-transitory recording medium.

Such a computer-readable recording medium includes an optical medium such as a CD-ROM (compact disc-ROM) and a magnetic recording medium such as a memory card. It is also possible to provide such programs by downloading them over a network.

The embodiment disclosed above should be construed in all respects as being illustrative and not being restrictive. The scope of the invention is defined by the claims rather than the above description, and it is intended that the scope of the invention includes all changes that are within the range of the claims and their equivalents.

For example, the method for calculating SBP and DBP for each beat and the configuration of the pressure pulse wave detection unit 100 are not limited to those described above and may be known ones as disclosed in Patent documents 1-4.

Although the blood pressure measuring instrument 200 measures living body information on the basis of pressure pulse waves detected directly from a wrist by the pressure pulse wave detection unit 100, a configuration may be employed in which a living body signal is detected indirectly as in known wrist sphygmomanometers.

More specifically, a configuration is possible in which pressure pulse waves are detected as a living body signal using pressure control unit for controlling a pressure that a cuff exerts on a wrist and a pressure sensor for detecting an inner pressure of the cuff and a blood pressure is measured by a known oscillometric method. Another configuration is possible in which a blood flow sound (Korotkoff sound) is detected as a living body signal using a microphone instead of a pressure sensor and living body information including a blood pressure and a pulse rate is measured from the blood flow sound.

As described above, the following items are disclosed in this specification.

The disclosed living body information measuring instrument is a wrist-mounted living body information measuring instrument having a living body information measuring unit for measuring living body information on the basis of a living body signal detected from a wrist, including a main body which forms a space into which the wrist can be inserted; a first display unit and a second display unit which are provided on an outer circumference of the main body; and a display control unit which performs display controls on the first display unit and the second display unit, wherein the first display unit and the second display unit are disposed such that the space exists on a straight line that connects a center of a display screen of the first display unit and a center of a display screen of the second display unit, and a center of the space is located between the center of the display screen of the first display unit and the center of the display screen of the second display unit in a direction that is perpendicular to a palm in a state that the living body information measuring instrument is attached to the wrist.

The disclosed living body information measuring instrument further includes a pressure detection unit; and a pressing unit which presses the pressure detection unit toward an artery that runs under a skin of the wrist, wherein the living body information measuring unit measures living body information on the basis of pressure pulse waves detected as a living body signal by the pressure detection unit in a state that the pressure detection unit is pressed toward the artery by the pressing unit.

In the disclosed living body information measuring instrument, the display control unit causes the first display unit and the second display unit to display information that is based on the living body information measured by the living body information measuring unit.

In the disclosed living body information measuring instrument, the center of the display screen of the first display unit is closer to the palm than the center of the space in the direction that is perpendicular to the palm; the center of the display screen of the second display unit is closer to a back of a hand than the center of the space in the direction that is perpendicular to the palm; and the display control unit causes the second display unit to display, as the information based on the living body information, information for notification of pulse rhythm that is measured on the basis of the pressure pulse waves as the living body signal, and causes the first display unit to display, as the information based on the living body information, blood pressure values that are measured on the basis of the pressure pulse waves.

In the living body information measuring instrument, the display control unit selectively performs a first control for displaying different pieces of information on the first display unit and the second display unit and a second control for displaying the same information on the first display unit and the second display unit.

In the disclosed living body information measuring instrument, the center of the display screen of the first display unit is closer to the palm than the center of the space in the direction that is perpendicular to the palm; the center of the display screen of the second display unit is closer to a back of a hand than the center of the space in the direction that is perpendicular to the palm; the first control is a control for causing the second display unit to display, as the information based on the living body information, information for notification of pulse rhythm that is measured on the basis of the pressure pulse waves as the living body signal, and causing the first display unit to display, as the information based on the living body information, blood pressure values that are measured on the basis of the pressure pulse waves; and the second control is a control for causing each of the first display unit and the second display unit to display, as the information based on the living body information, at least blood pressure values that are measured on the basis of the pressure pulse waves among the blood pressure values and a pulse rate that is measured on the basis of the pressure pulse waves.

In the disclosed living body information measuring instrument, the center of the display screen of the first display unit is closer to the palm than the center of the space in the direction that is perpendicular to the palm; the center of the display screen of the second display unit is closer to a back of a hand than the center of the space in the direction that is perpendicular to the palm; the living body information measuring instrument further includes a living body information acquiring unit which acquires living body information other than the living body information measured by the living body information measuring unit; and the information that the display control unit causes the first display unit to display includes the living body information acquired by the living body information acquiring unit.

## INDUSTRIAL APPLICABILITY

The invention can provide a living body information measuring instrument which allows both of a measurement-subject person and a third person to check measured living body information easily by giving a higher degree of freedom to the manner of display of measured living body information and enables consideration for preservation of the privacy of the measurement-subject person.

Although the invention has been described above using the particular embodiment, the invention is not limited to this embodiment. Various modifications are possible without departing from the technical concept of the disclosed invention.

The present application is based on Japanese Patent Application No. 2015-38728 filed on February 27, 2015, the disclosure of which is incorporated herein.

## DESCRIPTION OF SYMBOLS

100: Pressure pulse wave detection unit
101, 102: Body unit
103: Hinge
104, 105: Display unit
106: Belt
200: Blood pressure measuring instrument
T: Radius artery
H: Wrist
Ka: Space
1: Body
2: Air bag (pressing unit)
3: Flat plate member
5: Rotary member
5a: Biaxial rotating mechanism
6: Sensor unit
6a, 7a: Pressure sensor (pressure detecting element)
6b: Pressing surface
X, Y: Rotation axis
10: Rotational drive unit
11: Air bag drive unit
12: Control unit (display control unit, living body information measuring unit, living body information acquiring unit)
14: Manipulation unit
15: Memory
TB: Radius
K: Tendon
P: Pressing force
M: Pulse wave
105A: Icon

107: Curved display
110: Body

## Claims

1.  A wrist-mounted living body information measuring instrument having a living body information measuring unit for measuring living body information on the basis of a living body signal detected from a wrist, including:

    a main body which forms a space into which the wrist can be inserted;
    a first display unit and a second display unit which are provided on an outer circumference of the main body; and
    a display control unit which performs display controls on the first display unit and the second display unit,
    wherein the first display unit and the second display unit are disposed such that the space exists on a straight line that connects a center of a display screen of the first display unit and a center of a display screen of the second display unit, and a center of the space is located between the center of the display screen of the first display unit and the center of the display screen of the second display unit in a direction that is perpendicular to a palm in a state that the living body information measuring instrument is attached to the wrist.

2.  The living body information measuring instrument of Claim 1, further including:

    a pressure detection unit; and
    a pressing unit which presses the pressure detection unit toward an artery that runs under a skin of the wrist,
    wherein the living body information measuring unit measures living body information on the basis of pressure pulse waves detected as a living body signal by the pressure detection unit in a state that the pressure detection unit is pressed toward the artery by the pressing unit.

3.  The living body information measuring instrument of Claim 1 or 2,
    wherein the display control unit causes the first display unit and the second display unit to display information that is based on the living body information measured by the living body information measuring unit.

4.  The living body information measuring instrument of Claim 3,
    wherein the center of the display screen of the first display unit is closer to the palm than the center of the space in the direction that is perpendicular to the palm,

wherein the center of the display screen of the second display unit is closer to a back of a hand than the center of the space in the direction that is perpendicular to the palm, and

wherein the display control unit causes the second display unit to display, as the information based on the living body information, information for notification of pulse rhythm that is measured on the basis of the pressure pulse waves as the living body signal, and causes the first display unit to display, as the information based on the living body information, blood pressure values that are measured on the basis of the pressure pulse waves.

5. The living body information measuring instrument of Claim 3,

wherein the display control unit selectively performs a first control for displaying different pieces of information on the first display unit and the second display unit and a second control for displaying the same information on the first display unit and the second display unit.

6. The living body information measuring instrument of Claim 5,

wherein the center of the display screen of the first display unit is closer to the palm than the center of the space in the direction that is perpendicular to the palm,

wherein the center of the display screen of the second display unit is closer to a back of a hand than the center of the space in the direction that is perpendicular to the palm,

wherein the first control is a control for causing the second display unit to display, as the information based on the living body information, information for notification of pulse rhythm that is measured on the basis of the pressure pulse waves as the living body signal, and causing the first display unit to display, as the information based on the living body information, blood pressure values that are measured on the basis of the pressure pulse waves, and

wherein the second control is a control for causing each of the first display unit and the second display unit to display, as the information based on the living body information, at least blood pressure values that are measured on the basis of the pressure pulse waves among the blood pressure values and a pulse rate that is measured on the basis of the pressure pulse waves.

7. The living body information measuring instrument of any one of Claims 1 to 6,

wherein the center of the display screen of the first display unit is closer to the palm than the center of the space in the direction that is perpendicular to the palm,

wherein the center of the display screen of the second display unit is closer to a back of a hand than the center of the space in the direction that is perpendicular to the palm,

wherein the living body information measuring instrument further includes a living body information acquiring unit which acquires living body information other than the living body information measured by the living body information measuring unit, and

wherein the information that the display control unit causes the first display unit to display includes the living body information acquired by the living body information acquiring unit.

**FIG. 1**

**FIG. 2**

**FIG. 3**

**FIG. 4**

**FIG. 5**

*FIG. 6*

*FIG. 7*

**FIG. 8**

**FIG. 9**

*FIG. 10A*

*FIG. 10B*

POSITION OF
PRESSURE SENSOR

PRESSING
FORCE

A4

A3

A2

A5     A1

## FIG. 11A

POSITION OF
PRESSURE SENSOR

PRESSING
FORCE

A4     A1  A2   A3          A5

## FIG. 11B

*FIG. 12A*

*FIG. 12B*

*FIG. 12C*

*FIG. 13*

FIG. 14

*FIG. 15*

START

START INCREASING PRESSING FORCE —— S21

DETERMINE OPTIMUM PRESSURE SENSOR AND
OPTIMUM PRESSING FORCE ON THE BASIS OF
PRESSURE PULSE WAVES DETECTED BY
RESPECTIVE PRESSURE SENSORS 6a AND 7a —— S22

CANCEL PRESSING —— S23

PERFORM PRESSING
WITH OPTIMUM PRESSING FORCE —— S24

ACQUIRE PRESSURE PULSE WAVE
DETECTED BY OPTIMUM PRESSURE SENSOR —— S25

CALCULATE SBP AND DBP BY CALIBRATING
ACQUIRED PRESSURE PULSE WAVE
USING CALIBRATION DATA —— S26

CALCULATE PULSE RATE,
DISPLAY PULSE RATE AND PULSE RHYTHM. —— S27

S28
CONTINUOUS
NO    BLOOD PRESSURE
MEASUREMENT
ENDS ?

YES

END

110
85
♡60 ⌐104

15:45
2014/12/4 ⌐105
⊘ ⌐105A

# FIG. 16

110

85

♡ 60

15:45
2014/12/4

— 104

110

85

15:45
2014/12/4 ♡ 60

— 105

## FIG. 17

**FIG. 18**

# EP 3 263 023 A1

**INTERNATIONAL SEARCH REPORT**

International application No.

PCT/JP2016/055588

## A. CLASSIFICATION OF SUBJECT MATTER

*A61B5/022*(2006.01)i, *A61B5/02*(2006.01)i, *A61B5/0245*(2006.01)i

According to International Patent Classification (IPC) or to both national classification and IPC

## B. FIELDS SEARCHED

Minimum documentation searched (classification system followed by classification symbols)

A61B5/00-5/22

Documentation searched other than minimum documentation to the extent that such documents are included in the fields searched

| | | |
|---|---|---|
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| A | JP 2010-220949 A (Citizen Holdings Co., Ltd.), 07 October 2010 (07.10.2010), paragraphs [0026] to [0069] (Family: none) | 1-7 |
| A | WO 2012/018029 A1 (OMSI Co., Ltd.), 09 February 2012 (09.02.2012), paragraphs [0022] to [0094] (Family: none) | 1-7 |
| A | WO 2013/157393 A1 (Omron Healthcare Co., Ltd.), 24 October 2013 (24.10.2013), paragraphs [0027] to [0065] & US 2015/0105676 A1 paragraphs [0046] to [0084] & JP 2013-220177 A     & CN 104203087 A | 1-7 |

☐ Further documents are listed in the continuation of Box C.      ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 17 May 2016 (17.05.16) | 24 May 2016 (24.05.16) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**EP 3 263 023 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004113368 A
- JP H02261421 A
- JP H07124130 A
- JP H01242031 A
- JP 2010220949 A
- WO 2012018029 A
- JP 2015038728 A